# EUROPÄISCHE PATENTSCHRIFT

(11) **EP 1 733 028 B1**
(45) Veröffentlichungstag und Bekanntmachung des Hinweises auf die Patenterteilung: **15.08.2012**
(21) Anmeldenummer: 05715742.2
(22) Anmeldetag: 04.03.2005
(51) Int. Cl.: C12N 5/071

(54) **VERFAHREN ZUR HERSTELLUNG EINER BIOLOGISCHEN MATERIALZUSAMMENSETZUNG TIERISCHEN URSPRUNGS**
METHOD FOR THE PRODUCTION OF A BIOLOGICAL MATERIAL COMPOSITION HAVING AN ANIMAL ORIGIN
PROCEDE DE PRODUCTION D'UNE COMPOSITION DE MATIERES BIOLOGIQUES D'ORIGINE ANIMALE

(30) Priorität: 08.04.2004 DE 102004017484
(43) Veröffentlichungstag der Anmeldung: 20.12.2006
(73) Patentinhaber: Fraunhofer-Gesellschaft zur Förderung der angewandten Forschung e.V., 80686 München (DE)
(72) Erfinder: KRUSE, Charli, 23923 Herrnburg (DE); FUHR, Günter, 13187 Berlin (DE); ROHWEDEL, Jürgen, 23552 Lübeck (DE)
(74) Vertreter: Katzameyer, Michael
(86) Internationale Anmeldenummer: PCT/EP2005/002313
(87) Internationale Veröffentlichungsnummer: WO 2005/097975

(56) Entgegenhaltungen:
- WO-A-02/086107
- WO-A-2005/001072
- WO-A-2005/001073
- RAMIYA VIJAYAKUMAR K ET AL: "Reversal of insulin-dependent diabetes using islets generated in vitro from pancreatic stem cells" NATURE MEDICINE, NATURE AMERICA, NEW YORK, US, Bd. 6, Nr. 3, März 2000 (2000-03), Seiten 278-282, XP000864764 ISSN: 1078-8956
- MITAKA TOSHIHIRO: "Reconstruction of hepatic organoid by hepatic stem cells." JOURNAL OF HEPATO-BILIARY-PANCREATIC SURGERY. 2002, Bd. 9, Nr. 6, 2002, Seiten 697-703, XP002331148 ISSN: 0944-1166
- KRUSE, C. ET AL: "Pluripotency of adult stem cells derived from human and rat pancreas" APPLIED PHYSICS A: MATERIALS SCIENCE & PROCESSING , A79(7), 1617-1624 CODEN: APAMFC; ISSN: 0947-8396, 2004, XP002331149

## Beschreibung

Die Erfindung betrifft Verfahren zur Herstellung von biologischen Materialzusammensetzungen tierischen Ursprungs, insbesondere als Futter- oder Lebensmittel.

Die Produktion von Biomasse als Futtermittel zur Ernährung von Tieren ist eine der wichtigsten Herausforderungen der modernen Tierwirtschaft. Wichtige Probleme der konventionellen Produktion von Futtermitteln tierischen Ursprungs, insbesondere für Haustiere oder Nutztiere, bestehen erstens in der Verfügbarkeit biologischen Ausgangsmaterials, dessen Bereitstellung und Gewinnung einen großen wirtschaftlichen Aufwand darstellt und ethische Probleme verursacht, und zweitens in der Gefahr, dass Krankheitserreger in den Nahrungskreislauf eingeschleust und ggf. auf den Menschen übertragen werden.

Ähnliche Probleme bestehen auch bei der Produktion von Lebensmitteln tierischen Ursprungs zur Ernährung von Menschen. Die sog. Fleischproduktion (einschließlich Aufzucht, Tierhaltung und Schlachtung) erfolgt heute unter Bedingungen, die insbesondere für das Befinden von Säugetieren ethisch höchst problematisch sind.

Es besteht ein starkes Interesse an einer wachsenden Futter-und Lebensmittelproduktion auf der Grundlage tierischer Ausgangsmaterialien, um beispielsweise den Anforderungen des Bevölkerungswachstums und der Ernährung in Hungergebieten gerecht zu werden. Weder eine erweiterte Tierproduktion noch ein verstärkter Fischfang liefern hierzu dauerhafte Lösungen.

Die Aufgabe der Erfindung ist es, verbesserte Verfahren zur Herstellung einer biologischen Materialzusammensetzung tierischen Ursprungs, insbesondere als Futter- oder Lebensmittel, bereitzustellen, mit denen die Probleme der herkömmlichen Futter- oder Lebensmittelproduktion überwunden werden und die insbesondere ermöglichen, Futter- oder Lebensmittel zu produzieren, die in ihrer Zusammensetzung und Verwertbarkeit den bisher gewonnenen Tierprodukten gleichwertig oder identisch sind. Ein weiterer Gesichtspunkt der Aufgabe der Erfindung ist es, eine entsprechende biologische Materialzusammensetzung für Futter- oder Ernährungszwecke bereitzustellen, bei deren Produktion die Nachteile der herkömmlichen Tierproduktion vermieden werden, d.h. keine Organismen gezüchtet, gemästet und zu Fleischprodukten verarbeitet werden müssen.

Diese Aufgaben werden durch Verfahren mit den Merkmalen des Anspruchs 1 gelöst. Vorteilhafte Ausführungsformen und Anwendungen der Erfindung ergeben sich aus den abhängigen Ansprüchen.

Die oben genannte Aufgabe wird gelöst durch eine Aggregation von Stammzellen, die aus differenziertem exokrinen Drüsengewebe eines tierischen (nicht-menschlichen) Organismus isoliert wurden, zu dreidimensionalen Zellverbänden und eine anschließende Präparation der gewünschten Materialzusammensetzung auf der Grundlage der Zellverbände gelöst. Die Zellverbände werden im Folgenden auch als organoide Körper bezeichnet. Ein besonderer Vorteil dieses Verfahrens besteht in der kompletten Abkopplung der Biomasseproduktion von der herkömmlichen Tierproduktion, wobei dennoch der hergestellten Materialzusammensetzung in Abhängigkeit von dem tierischen Organismus, aus dem die Stammzellen isoliert wurden, und konkreten Differenzierungsbedingungen eine stoffliche, strukturelle und insbesondere geschmackliche Eigenart aufgeprägt werden kann.

Die Erfinder haben festgestellt, dass die aus dem exokrinen Drüsengewebe isolierten adulten Stammzellen pluripotent sind und eine hohe Teilungsfähigkeit und ein starkes Wachstum zeigen. Die Biomasseproduktion erfolgt nicht auf natürlichem Weg durch das Wachstum des Tieres, sondern synthetisch insbesondere bei der Präparation der Materialzusammensetzung in so genannten in-vitro Laborkulturen.

In den nachveröffentlichten Druckschriften WO 2005/001072 und WO 2005/001073 wird bereits die Aggregation solcher pluripotenten adulten Stammzellen zu organoiden Körpern beschrieben, nicht jedoch die weiteren speziellen Verfahrenschritte des hier beanspruchten Verfahrens.

Ramiya et al. beschreiben in Nature Medicine, Bd. 6, Nr. 3, S. 278-282 (2000), die Bildung von insulinproduzierenden Langerhans'schen Inseln aus adulten Stammzellen des Pankreas. Bei diesen Stammzellen handelt es sich jedoch nicht um pluripotente adulte Stammzellen, die zu Zellen aller drei Keimblätter differenzieren können.

WO 02/086107 A offenbart ein Verfahren zur Kultivierung von embryonalen Stammzellen und deren Aggregation zu "embryonalen Körpern" (embryoid bodies).

Das erfindungsgemäß verwendete exokrine Drüsengewebe kann von einem nicht-humanen erwachsenen Organismus, juvenilen Organismus oder fötalen Organismus, vorzugsweise einem postnatalen Organismus, stammen. Der Begriff "adult", wie in der vorliegenden Anmeldung verwendet, bezieht sich somit auf das Entwicklungsstadium des Ausgangsgewebes und nicht auf dasjenige des Donororganismus, aus dem das Gewebe stammt. "Adulte" Stammzellen sind nicht-embryonale Stammzellen.

Vorzugsweise wird das exokrine Drüsengewebe aus einer Speicheldrüse, Tränendrüse, Talgdrüse, Schweißdrüse, aus Drüsen des Genitaltrakts, einschließlich Prostata, oder aus gastrointestinalem Gewebe, einschließlich Pankreas, oder sekretorischem Gewebe der Leber isoliert. In einer stark bevorzugten Ausführungsform handelt es sich dabei um acinäres Gewebe. Ganz besonders bevorzugt stammt das acinäre Gewebe aus dem Pankreas, der Ohrspeicheldrüse oder Unterkieferspeicheldrüse.

Ein wesentlicher Vorteil des erfindungsgemäßen Verfahrens besteht darin, dass die Stammzellen aus lebenden Donortieren, z. B. aus Speicheldrüsen von Schweinen gewonnen werden können, ohne dass das Donortier geschlachtet werden muss. Dies ist sowohl unter ethischen Gesichtspunkten als auch mit Blick auf die Möglichkeit der weiteren Beobachtung des Donortieres hinsichtlich eventueller Krankheiten von besonderem Vorteil.

Allgemein kann erfindungsgemäß die Materialzusammensetzung unmittelbar aus den organoiden Körpern gebildet werden. Gemäß einer bevorzugten Ausführungsform der Erfindung ist jedoch vorgesehen, dass die organoiden Körper nach der Aggregation, z. B. in einer Suspensionskultur oder einer Adhäsionskultur unter Nährstoffversorgung zu größeren Körpern wachsen, die im Folgenden als Gewebekörper bezeichnet werden. Die Erfinder haben festgestellt, dass die aus den exokrinen Drüsen isolierten Stammzellen organoide Körper bilden, die bei Nährstoffversorgung ein starkes Wachstum zu Gewebekörpern mit Durchmessern von einigen Millimetern oder darüber zeigen. Die Präparation der Materialzusammensetzung auf der Grundlage der Gewebekörper besitzt den Vorteil einer erhöhten Effektivität der Biomaterialproduktion. Die organoiden Körper enthalten dabei keine Organe oder ein funktionsfähiges Nervensystem, sondern bestehen aus Gewebeschichten verschiedener Zellzusammensetzungen.

Wenn gemäß einer ersten Variante der Erfindung die Aggregation der Stammzellen und/oder das Wachstum der organoiden Körper in einem Kulturmedium ohne eine Zusatz erfolgen, der die Differenzierung der Zellen des organoiden Körpers beeinflussen könnte, wachsen vorteilhafterweise organoide Körper oder Gewebekörper mit einer Zusammensetzung von verschiedenen Zelltypen, die gemeinsam zur Struktur und Verwendbarkeit, insbesondere dem Geschmack der gewünschten Materialzusammensetzung beitragen. Wenn die Aggregation und/oder das Wachstum hingegen in Gegenwart von mindestens einem die Differenzierung beeinflussenden Zusatz erfolgt, können organoide Körper oder Gewebekörper mit einem oder mehreren bevorzugten Zelltypen gebildet werden. Es können verschiedene Zelltypen kombiniert werden, die von verschiedenen Donortieren stammen, um bestimmte Nähr- oder Geschmackseigenschaften zu erzielen.

Gemäß der Erfindung werden die organoiden Körper oder die aus diesen gewachsenen Gewebekörper zu einem Komposit (Formkörper) zusammengefügt. Wenn die Zellen im Komposit leben, kann vorteilhafterweise ein weiteres Wachstum des Komposits bis zu der gewünschten Größe als Futter- oder Lebensmittel erfolgen. Wenn hingegen die Zellen im Komposit abgestorben sind und nicht weiter wachsen, können sich Vorteile für die weitere Bearbeitung der Materialzusammensetzung ergeben.

Gemäß bevorzugten Varianten der Erfindung umfasst daher die Bildung des Komposits einen oder mehrere der folgenden Schritte. Erstens kann ein Zusammenwachsen der beteiligten organoiden Körper oder Gewebekörper zum Komposit vorgesehen sein, das dann vorteilhafterweise weiter wachsen kann. Zweitens kann ein adhärentes Anhaften der beteiligten organoiden Körper oder Gewebekörper vorgesehen sein, wobei auch in diesem Fall ein weiteres Wachstum vorgesehen sein kann. Durch ein Zusammenpressen der organoiden Körper oder Gewebekörper kann ein Verdichten des Formkörpers und damit eine bestimmte Struktur erzielt werden. Schließlich kann eine Übertragung der organoiden Körper oder Gewebekörper auf ein biokompatibles Trägersubstrat aus einem verdaubaren Material vorgesehen sein, auf dem eine weitere Präparation oder Formung der Materialzusammensetzung erfolgt.

Gemäß einer weiteren bevorzugten Ausführungsform der Erfindung kann das Komposit einer Formgebung durch eine Prägeeinrichtung unterzogen werden. In diesem Fall können sich Vorteile für die Präsentation der Futter- oder Lebensmittel für deren Verbrauch ergeben. Die Prägeeinrichtung kann z. B. ein besonders geformtes Kultivierungssubstrat, wie z. B. das Trägersubstrat, eine Stempeleinrichtung mit einer Prägeoberfläche oder einen flexiblen Beutel umfassen, der eine Aufnahmehülle bildet.

Gemäß einer Modifikation des erfindungsgemäßen Verfahrens kann bei der Präparation der Materialzusammensetzung eine Struktureinstellung vorgesehen sein. Dadurch kann die Materialzusammensetzung mit einer gewünschten Konsistenz, wie z. B. fest, zäh, locker oder dgl. gebildet werden, was insbesondere für die Verwendung als Lebensmittel von Vorteil sein kann. Die Struktureinstellung kann bspw. durch die genannte mechanische Verdichtung erfolgen. Alternativ wird insbesondere, wenn die Gewebekörper elektrisch anregbare Muskelzellen enthalten, eine Struktureinstellung durch eine Einwirkung elektrischer Felder realisiert. Das Komposit wird durch ein elektrisches Feld angeregt, so dass die Muskelzellen eine Struktur bilden, die der von Muskelfleisch ähnlich ist.

Besonders bevorzugt werden die organoiden Körper einer Differenzierung unterzogen, die zu wenigstens einem der folgenden Zelltypen führt, die Muskelzellen, Bindegewebszellen, Fettzellen und Enzym-produzierende Zellen umfassen. In diesem Fall enthalten die organoiden Körper oder entsprechende Gewebekörper vorrangig den differenzierten Zelltyp. Die Differenzierung kann allgemein durch den Zusatz an sich bekannter Differenzierungsfaktoren erfolgen. Besonders bevorzugt erfolgt jedoch bei der Kultivierung der organoiden Körper oder dem Wachstum der Gewebekörper ein Zusatz von bereits differenzierten, insbesondere autologen Zellen, die die weitere Differenzierung im organoiden Körper oder Gewebekörper auslösen oder beeinflussen.

Wenn die biologische Materialzusammensetzung aus verschiedenen Gruppen organoider Körper oder Gewebekörper, die jeweils verschieden differenziert sind, präpariert wird, können sich Vorteile für die weitere Verwertung der Materialzusammensetzung, insbesondere in Bezug auf die Nährstoffzusammensetzung, die Struktur und den Geschmack ergeben. Alternativ oder ergänzend können der Materialzusammensetzung erfindungsgemäß Geschmacksstoffe zugesetzt werden, wie sie an sich aus der Lebensmitteltechnik bekannt sind.

Weitere Einzelheiten und Vorteile der Erfindung werden im Folgenden unter Bezug auf die beigefügten Zeichnungen erläutert. Es zeigen:
- Figur 1:: ein Flussdiagramm zur Illustration einer Ausführungsform des erfindungsgemäßen Verfahrens,
- Figur 2:: eine Illustration der Bildung primärer organoider Körper,
- Figur 3:: ein Flussdiagramm zur Illustration mit weiteren Einzelheiten des erfindungsgemäßen Verfahrens,
- Figur 4:: eine Darstellung der Bildung sekundärer organoider Körper,
- Figur 5:: eine Photographie eines Gewebekörpers,
- Figur 6:: ein Flussdiagramm mit optionalen zusätzlichen Schritten des erfindungsgemäßen Verfahrens,
- Figur 7:: eine Schemadarstellung zur Illustration der Kultivierung organoider Körper,
- Figur 8:: eine Schemadarstellung mit weiteren Einzelheiten einer Ausführungsform des erfindungsgemäßen Verfahrens,
- Figur 9:: Illustrationen der Präparationsschritte gemäß verschiedenen Ausführungsformen der Erfindung, und
- Figur 10:: eine Illustration einer elektrischen Struktureinstellung an einer erfindungsgemäßen erhaltenen Materialzusammensetzung.

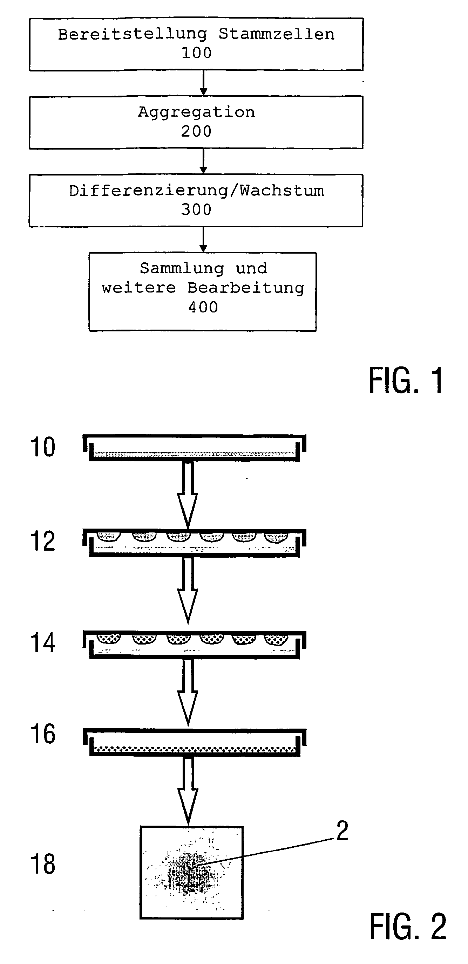

Zur erfindungsgemäßen Erzeugung einer Zusammensetzung aus biologischen Zellen und/oder Gewebe erfolgen gemäß Figur 1 zunächst eine Bereitstellung der von einem Donororganismus isolierten Stammzellen (Schritt 100), anschließend deren Aggregation zu organoiden Körpern (Schritt 200), die einer Differenzierung und/oder einem Wachstum (Schritt 300) und schließlich einer Sammlung und weiteren Bearbeitung (Schritt 400) unterzogen werden. Obwohl die Schritte 100 und 200 im Wesentlichen den erfindungsgemäßen Aggregationsschritt umfassen, während die Schritte 300 und 400 im Wesentlichen den erfindungsgemäßen Präparationsschritt repräsentieren und diese beiden Komplexe im Folgenden getrennt erörtert werden, wird betont, dass Teilschritte der Präparation bereits im Rahmen der Aggregation erfolgen und umgekehrt Teilschritte der Aggregation erst im Rahmen der Präparation realisiert werden können. So kann bspw. die Differenzierung der organoiden Körper bereits im Rahmen der Aggregation beginnen, oder es können isolierte Stammzellen mit bereits vordifferenzierten organoiden Körpern aggregieren.

Die im Folgenden erläuterten Ausführungsformen der Erfindung beziehen sich beispielhaft auf die Erzeugung der biologischen Materialzusammensetzung aus Stammzellen, die Ratten oder Schweinen entnommen wurden. Die Umsetzung der Erfindung ist jedoch nicht auf diese Tierarten beschränkt. Vielmehr können die entsprechenden Verfahren an allen nicht-menschlichen Organismen mit differenzierten exokrinen Drüsen mit acinärem Gewebe realisiert werden, so z. B. mit Fischen, die acinäres Gewebe an der Pankreasdrüse besitzen, oder Säugetieren, wie zum Beispiel Rindern oder Schafen.

### (I) Aggregation von Stammzellen aus differenziertem exokrinen Drüsengewebe eines tierischen Organismus zu organoiden Körpern und/oder Gewebekörpern

Entsprechend dem in Figur 2 dargestellten Schema wird zur Gewinnung der Zellen acinäres Gewebe - bevorzugt einer Speicheldrüse oder der Bauchspeicheldrüse (Pankreas) - mechanisch und enzymatisch zerkleinert in Kultur genommen (Schritt 10 in Figur 2). Entgegen den Angaben von Bachem et al., Gastroenterol. 115:421-432 (1998), und Grosfils et al., Res. Comm. Chem. Pathol. Pharmacol. 79:99-115 (1993), werden keine Gewebeblöcke kultiviert, aus denen Zellen auswachsen sollen, sondern unter der Bedingung, dass die Zellverbände der Acini weitestgehend intakt bleiben, das Gewebe stärker zerkleinert.

Über mehrere Wochen werden diese Zellen und Zellverbände in Kulturgefäßen kultiviert. Alle 2 bis 3 Tage wird das Medium gewechselt, wobei alle differenzierten Zellen entfernt werden. Bei den in Kultur persistierenden Zellen handelt es sich um undifferenzierte Zellen mit uneingeschränkter Teilungsfähigkeit.

Ähnliche Zellen sind unter gleichen Bedingungen aus dem Pankreas isoliert und beschrieben und als eine Art Myofibroblasten bzw. pankreatische Sternzellen bezeichnet worden (Bachem et al., 1998). Im Gegensatz zu den Zellen der vorliegenden Erfindung konnte eine uneingeschränkte Teilungsfähigkeit jedoch nicht beobachtet werden. Weiterhin konnten diese Zellen auch nicht unbegrenzt passagiert werden, ohne an Vitalität zu verlieren.

In einem zweiten Schritt (12) werden ungefähr 400 bis 800 Zellen in je 20 µl Medium in hängenden Tropfen kultiviert. Dazu werden die Tropfen auf Deckel von bakteriologischen Petrischalen gegeben, umgedreht und über die mit Medium gefüllte Petrischale gelegt, so dass die Tropfen nach unten hängen.

Durch diese Art der Kultivierung bilden sich innerhalb von 48h die als organoide Körper bezeichneten Zellaggregate (14), die für ungefähr 6 Tage in eine Suspensionskultur umgesetzt werden (16). Die Teilansicht (18) aus Figur 2 zeigt eine mikroskopische Aufnahme eines solchen organoiden Körpers 2.

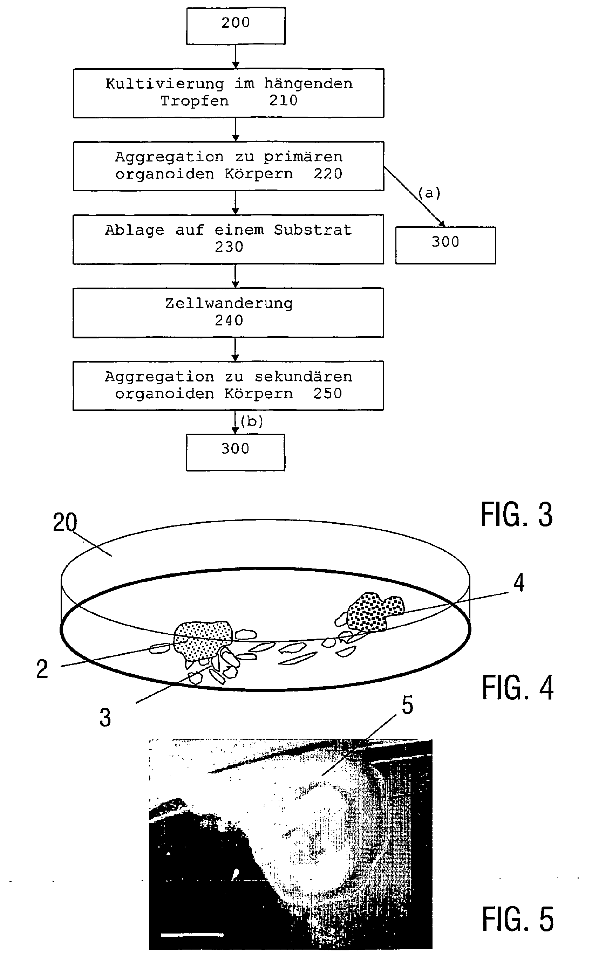

In Figur 3 sind die verschiedenen Möglichkeiten, organoide Körper als Ausgangsmaterialien für die weitere Präparation bereitzustellen, mit weiteren Einzelheiten zusammengestellt. Nach der oben beschriebenen Kultivierung der isolierten Stammzellen im hängenden Tropfen (Schritt 210) erfolgt die Aggregation zu den so genannten primären organoiden Körpern (Schritt 220), die direkt der Differenzierung und dem Wachstum (Schritt 300 in Figur 1) unterzogen werden können.

Alternativ erfolgt zunächst die Ablage der primären organoiden Körper auf einem Substrat zur Schaffung einer Adhäsionskultur (siehe auch Figur 4). Die Erfinder haben festgestellt, dass die in Suspensionskultur wachsenden primären organoiden Körper in Adhäsionskultur neue organoide Körper bilden können. Auf dem Substrat folgt durch eine Zellwanderung die Bildung einer Monoschicht (Schritt 240) und aus dieser die Aggregation zu den so genannten sekundären organoiden Körpern (Schritt 250). Diese können wiederum direkt der weiteren Differenzierung und/oder dem Wachstum (Schritt 300) unterzogen werden.

Die Bildung der sekundären organoiden Körper ist auch in Figur 4 illustriert. Die primären organoiden Körper 2 bilden auf dem Substrat der Adhäsionskultur, wie z. B. auf dem Boden der Kulturschale 20, durch ein Wandern oder Wachsen von Zellen 3 zunächst eine Monoschicht, aus der dann die sekundäre organoide Körper 4 herauswachsen. Mit der Kultivierung der primären organoiden Körper 2 zu sekundären organoiden Körpern 4 wird eine weitere Vervielfältigung des Biomaterials geschaffen.

Aus jedem der primären oder sekundären organoiden Körper 2, 4 können bei weiterer Kultivierung Gewebekörper wachsen. Figur 5 zeigt beispielhaft einen in der Adhäsionskultur gewachsenen Gewebekörper 5 (der weiße Strich entspricht einer Länge von 2 mm im Original).

### Ausführungsbeispiele für die Isolierung und Aggregation von Stammzellen

In den folgenden, nicht-beschränkenden Beispielen wird die Isolierung und Aggregation von Stammzellen näher erläutert.

Die allgemeinen Arbeitsanweisungen, wie sie für Verfahren zur Kultivierung von tierischen Zellen und insbesondere von Säugetierzellen gebräuchlich sind, sind zu beachten. Eine sterile Umgebung, in der das Verfahren durchgeführt werden soll, ist - auch wenn hierzu keine weitere Beschreibung erfolgt - in jedem Fall einzuhalten. Folgende Puffer und Medien wurden verwendet:

| HEPES-Stammlösung (pH 7.6) | 2,3 83 g HEPES auf 100 ml A. *bidest.* | |
|---|---|---|
| HEPES-Eagle-Medium (pH 7,4) | 90 ml Modified Eagle Medium (MEM) | |
| | 10 ml HEPES-Stammlösung | |
| Isolationsmedium (pH 7,4) | 32 ml HEPES-Eagle-Medium | |
| | 8 ml 5% BSA in A. bidest. | |
| | 300 µl 0,1 M CaC12 | |
| | 100 µl Trasylol (200.000 KIE) | |
| Digestionsmedium (pH 7,4) | 20 ml Isolationsmedium | |
| | 4 ml Kollagenase (Kollagenase NB 8 von Serva) | |

| Inkubationsmedium | Dulbecco's Modified Eagle Medium (DMEM) | |
|---|---|---|
| Nährmedium | Dulbecco's Modified Eagle Medium (DMEM) | |
| | DMEM | + 4500 mg/l Glucose |
| | | + L-Glutamin |
| | | - Pyruvat |
| | + 20 % FKS (inaktiviert) + 1 ml/100 ml Pen/Strep | |
| | (10000 E/10000 µg/ml) | |
| | oder | |
| | DMEM + 10 % Eigenplasma + 1 ml/100 ml | |
| | Pen/Strep, | |
| | vor Gebrauch auf 37°C erwärmen | |

| | | |
|---|---|---|
| Differenzierungsmedium | 380 ml DMEM | |
| | 95 mol 30 min bei 54 °C inaktiviertes FKS | |
| | 5 ml Glutamin (GIBCO BRL) | |
| | 5 ml (3,5 µl ß-Mercaptoethanol auf 5 ml PBS) | |
| | 5 ml Non-essential amino acids (GIBCO BRL) | |
| | 5 ml Penicillin/Streptomycin (GIBCO BRL) | |
| | (10000 E/10000 µg/ml) | |

Statt fötalem Kalbserum (FKS) im Nährmedium und Differenzierungsmedium kann gegebenenfalls auch Eigenplasma oder, weniger bevorzugt, Eigenserum des Gewebedonors verwendet werden.

Das Nährmedium kann als Basismedium statt des verwendeten DMEM-Mediums auch ein anderes für die Kultivierung von eukaryotischen Zellen, insbesondere Säugerzellen, bekanntes, geeignetes Basismedium enthalten, in dem die differenzierten Zellen absterben und sich die gewünschten Stammzellen vermehren. Auch Isolationsmedium, Inkubationsmedium und Differenzierungsmedium können ein anderes übliches und geeignetes Basismedium enthalten.

Die folgenden Beispiele 1 und 2 beschreiben detailliert zwei Arbeitsprotokolle zur Isolierung und Kultivierung adulter pluripotenter Stammzellen aus acinärem Gewebe des Pankreas. Beispiel 3 beschreibt ein entsprechendes Protokoll für die Isolierung aus acinärem Gewebe der Speicheldrüse.

### BEISPIEL 1

### 1. Präparation des Gewebes und Isolierung der Zellen

In den *Ductus pancreaticus* von 2-3 Jahre alten Ratten werden mittels einer Spritze und einer stumpfen Kanüle bei der Ratte 10 ml Digestionsmedium langsam und luftblasenfrei injiziert. Das gesamte Pankreas wird dadurch aufgeblasen und kann so besser herauspräpariert werden. Das Pankreas wird dann in ein Becherglas überführt und weitere 5 ml Digestionsmedium dazugegeben. Nachdem man das Fettgewebe und Lymphknoten entfernt hat, wird das Gewebe im Becherglas mit einer feinen Schere sehr fein zerkleinert, oben schwimmendes Fettgewebe abgesaugt und die Suspension wird abschließend 1 min mit Carbogen begast (wenn nötig wiederholen) und für 20 min bei 37 °C mit Alufolie bedeckt in einem Schüttler bei 200 Zyklen/min inkubiert. Danach saugt man das Medium vorsichtig ab, zerkleinert erneut mit einer Schere das Gewebe und wäscht die Gewebestücke zweimal mit je 10 ml Isolationsmedium und gibt wieder 5 ml Digestionsmedium zum Gewebe hinzu.

Nach erneutem Begasen mit Carbogen für etwa 1 min und Inkubation für 15 min bei 37 °C in einem Schüttler bei 200 Zyklen/min werden die Gewebestücke durch sukzessives Aufziehen in jeweils einer 10 ml, 5ml, 2 ml und 1 ml Glaspipette zerkleinert und durch einlagiges Filtergewebe gepresst. Die so vereinzelten Zellen werden nun fünfmal in Inkubationsmedium gewaschen (37 °C), mit Carbogen begast und jedes Mal 5 min bei 90 g zentrifugiert. Das zuletzt erhaltene Pellet wird in Inkubationsmedium resuspendiert, begast und auf Gewebekulturschalen verteilt.

### 2. Kultivierung der Zellen

Die Gewebekulturschalen mit den isolierten Zellen werden im Brutschrank bei 37°C und 5 % CO₂ kultiviert. Alle 2-3 Tage wird das Medium gewechselt. Dabei werden alle differenzierten Zellen entfernt.

Am siebenten Tag in Kultur werden die Zellen mit einer Lösung bestehend aus 2 ml PBS, 1 ml Trypsin und 2 ml Inkubationsmedium passagiert. Dabei lösen sich die Zellen vom Boden der Kulturschale. Die Zellsuspension wird 5 Minuten zentrifugiert, der Überstand abgesaugt und die Zellen in 2 ml Inkubationsmedium resuspendiert, auf eine mittlere Zellkulturflasche überführt und 10 ml Inkubationsmedium dazugegeben. Der Medienwechsel erfolgt alle drei Tage.

Am vierzehnten Tag in Kultur werden die Zellen erneut, aber diesmal mit 6 ml PBS, 3 ml Trypsin und 6 ml Inkubationsmedium, passagiert. Die Zellsuspension wird 5 Minuten zentrifugiert, der Überstand abgesaugt und die Zellen in 6 ml Inkubationsmedium resuspendiert, auf 3 mittlere Zellkulturflaschen überführt und jeweils 10 ml Inkubationsmedium dazugegeben.

Die Zellen werden weiter kultiviert und so oft passagiert und ausgesät, bis die Zellen einen semikonfluenten bis konfluenten Zustand erreichen.

### BEISPIEL 2

Pankreas-Acini wurden von männlichen Sprague-Dawley-Ratten (20-300 g) erhalten, die narkotisiert (CO₂) und über die dorsale Aorta ausgeblutet worden waren. Eine Kanüle wurde trasduodenal in den Pankreasgang eingeführt und dem Pankreas wurde von hinten 10 ml Digestionsmedium, enthaltend HEPES-Eagle-Medium (pH 7,4), 0,1 mM HEPES-Puffer (pH, 7,6), 70% (Vol./Vol.) modifiziertes Eagle-Medium, 0,5 % (Vol./Vol.) Trasylol (Bayer AG, Leverkusen, Deutschland), 1 % (Gew./Vol.) Rinderserumalbumin), 2,4 mM CaCl₂ und Kollagenase (0,63 P/mg, Serva, Heidelberg, Deutschland) injiziert.

Vor der Entfernung wurde der Pankreas von anhaftendem Festgewebe, Lymphknoten und Blutgefäßen teilweise befreit. Dann wurde gesundes Pankreasgewebe in Digestionsmedium abgeholt (bei 20 °C, geringerer Stoffwechsel), das Pankreasgewebe mit einer Schere sehr fein zerkleinert, oben schwimmendes Fettgewebe abgesaugt und die Gewebesuspension mit Carbogen (Messer, Krefeld, Deutschland) begast, ohne dass die Düse in das Medium mit den Zellen gelangt (Verringerung von mechanischem Streß) und damit auf pH 7,4 eingestellt. Danach wurde die Suspension in einem 25-ml-Erlenmeyerkolben (mit Alufolie bedeckt) unter konstantem Schütteln (150-200 Zyklen pro Minute) bei 37 °C in 10 ml Digestionsmedium inkubiert. Nach 15-20 Minuten wurde das oben schwimmende Fett und das Medium abgesaugt und das Gewebe wurde erneut zerkleinert und mit Medium ohne Kollagenase gespült (Vorgang mindestens zweimal wiederholen, vorzugsweise solange bis Zellfraktion transparent), worauf Digestionsmedium zugegeben und erneut etwa 1 Minute lang mit Carbogen begast wurde. Es folgte wiederum eine Digestion mit Kollagenase für 15 Minuten bei 37°C im Schüttler unter Verwendung desselben Puffers. Nach der Digestion wurden die Acini durch sukzessives Hochziehen und Ausstossen durch 10 ml-, 5 ml und 2 ml-Glaspipetten mit engen Öffnungen dissoziiert und durch ein einlagiges Nylonsieb (Polymon PES-200/45, Angst & Pfister AG, Zürich, Schweiz) mit einer Maschengröße von etwa 250 µm filtriert. Die Acini wurden zentrifugiert (bei 37°C und 600-800 UpM in einer Beckman-GPR-Zentrifuge, entspricht etwa 90 g) und weiter gereinigt durch Waschen in Inkubationsmedium, enthaltend 24,5 mM HEPES (pH 7,5), 96 mM NaCl, 6 mM KCl, 1 mM MgCl₂, 2,5 mM NaH₂PO₄, 0, mM CaCl₂, 11,5 mM Glucose, 5 mM Natriumpyruvat, 5 mM Natriumglutamat, 5 mM Natriumfumarat, 1 % (Vol./Vol.) modifiziertes Eagle-Medium, 1 % (Gew./Vol.) Rinderserumalbumin, mit Carbogen äquilibriert und auf pH 7,4 eingestellt. Die Waschprozedur (Zentrifugation, Absaugen, Resuspension) wurde fünfmal wiederholt. Soweit nicht anders angegeben, wird bei der obigen Isolierung bei etwa 20 °C gearbeitet.

Die Acini wurden in Inkubationsmedium resuspendiert und bei 37°C in einer angefeuchten Atmosphäre mit 5 % CO₂ kultiviert. Das acinäre Gewebe starb dabei schnell (innerhalb von zwei Tragen) ab und die sterbenden differenzierten Zellen lösten sich dabei von den benachbarten Zellen, ohne diese zu schädigen (schonende Isolierung), die nicht absterbenden Stammzellen sanken zu Boden und hefteten sich an. Die differenzierten Acinizellen sind dazu nicht in der Lage. Das Inkubationsmedium wurde am zweiten oder dritten Tag nach dem Aussäen erstmals gewechselt, wobei ein Großteil der frei schwimmenden Acini und acinären Zellen entfernt wurde. Zu diesem Zeitpunkt hatten sich die ersten Stammzellen bzw. deren Vorläufer am Boden festgesetzt und begannen sich zu teilen. Der Mediumwechsel wurde danach an jedem dritten Tag wiederholt und differenzierte acinäre Pankreaszellen wurden bei jedem Mediumwechsel entfernt.

Am siebenten Tag in Kultur wurden die Zellen mit einer Lösung bestehend aus 2 ml PBS, 1 ml Trypsin (+ 0,05 % EDTA) und 2 ml Inkubationsmedium passagiert. Dabei lösen sich die Zellen vom Boden der Kulturschale. Die Zellsuspension wurde 5 Minuten bei etwa 1000 UpM (Beckmann GPR-Zentrifuge) zentrifugiert, der Überstand abgesaugt und die Zellen in 2 ml Inkubationsmedium resuspendiert, auf eine mittlere Zellkulturflasche überführt und 10 ml Inkubationsmedium dazugegeben.

Am vierzehnten Tag in Kultur wurden die Zellen erneut, aber diesmal mit 6 ml PBS, 3 ml Trypsin/EDTA und 6 ml Inkubationsmedium, passagiert. Die Zellsuspension wird 5 Minuten bei 1000 UpM zentrifugiert, der Überstand abgesaugt und die Zellen in 6 ml Inkubationsmedium resuspendiert, auf 3 mittlere Zellkulturflaschen überführt und jeweils 10 ml Inkubationsmedium dazugegeben.

Am Tag 17 erfolgte ein drittes Passagieren auf insgesamt 6 mittlere Zellkulturflaschen und am Tag 24 ein viertes Passagieren auf insgesamt 12 mittlere Zellkulturflaschen. Spätestens jetzt waren alle primären Zellen bis auf die Stammzellen aus der Zellkultur entfernt.

Die Stammzellen können weiter kultiviert werden und so oft passagiert und ausgesät wie gewünscht. Das Aussäen erfolgt vorzugsweise jeweils in einer Dichte von 2-4 x 10⁵ Zellen/cm² in Inkubationsmedium.

### BEISPIEL 3

Die Isolierung und Kultivierung aus exokrinem Gewebe der Ohrspeicheldrüse erfolgte analog dem Pankreas-Protokoll mit den folgenden Abweichungen:
1. Das exokrine Gewebe der Ohrspeicheldrüse war eine Mischung von acinärem Gewebe und tubulösem Gewebe.
2. Nachdem Speicheldrüsen weniger Proteasen und Amylasen als Pankreas enthalten, ist es möglich, das Speicheldrüsengewebe vor der Aufarbeitung einige Zeit im Kühlschrank bei etwa 4°C aufzubewahren, ohne dass das Gewebe zu sehr geschädigt wird. Im konkreten Beispielsfall betrug die Aufbewahrungszeit 15 h und brachte keine nachteiligen Folgen für die Isolierung der gewünschten Stammzellen mit sich.

Die folgenden Beispiele 4 und 5 beschreiben detailliert zwei Arbeitsprotokolle zur Herstellung von organoiden Körpern und differenzierten Zellen.

### BEISPIEL 4

Die undifferenzierten Zellen werden mit einer Lösung aus 10 ml PBS, 4 ml Trypsin, 8 ml Differenzierungsmedium abtrypsiniert und 5 Minuten abzentrifugiert. Das resultierende Pellet wird so in Differenzierungsmedium resuspendiert, dass sich eine Verdünnung von 3000 Zellen je 100 µl Medium einstellt. Anschließend werden die Zellen nochmals gut mit einer 3 ml Pipette suspendiert.

Von bakteriologischen Petrischalen, die vorher mit jeweils 15 ml PBS (37 °C) pro Platte beschichtet worden sind, wird der Deckel abgenommen und umgedreht. Mit Hilfe einer automatischen Pipette werden auf einen Deckel ca. fünfzig 20 µl Tropfen gegeben. Der Deckel wird dann schnell umgedreht und auf die mit Differenzierungsmedium gefüllte Petrischale gegeben, sodass die Tropfen nach unten hängen. Die Petrischalen werden anschließend vorsichtig in den Brutschrank gestellt und für 48 h inkubiert.

Daraufhin werden die in den hängenden Tropfen aggregierten Zellen, die hier organoide Körper genannt werden, aus jeweils vier Deckeln in je eine bakteriologische Petrischale mit 5 ml Inkubationsmedium mit 20 % FKS überführt und für weitere 96 h kultiviert.

Die organoide Körper werden nun vorsichtig mit einer Pipette aufgesammelt, und in mit 0,1 % Gelatine beschichtete Zellkulturgefäße mit Differenzierungsmedium überführt. In einer besonders bevorzugten Ausgestaltung des Verfahrens verwendet man als Kulturgefäß mit 0,1 % Gelatine beschichtete 6 cm Petrischalen, in die 4 ml Differenzierungsmedium vorgelegt wurde und die anschließend mit je 6 organoiden Körpern beschickt werden. Ein weiteres bevorzugtes Kulturgefäß sind mit 0,1 % Gelatine beschichtete Chamber Slides, in die 3 ml Differenzierungsmedium vorgelegt wurde und die anschließend mit je 3-8 ,organoiden Körpern beschickt werden. Daneben können auch 24-Mulden-Mikrotiterplatten verwendet werden, die mit 0,1 % Gelatine beschichtet wurden und in die je 1,5 ml pro Mulde Differenzierungsmedium vorgelegt wurde und anschließend mit je 4 organoiden Körpern beschickt werden.

Derart kultiviert, ist die Differenzierungsfähigkeit der Zellen in den organoide Körper aktiviert und die Zellen differenzieren sich in Zellen der drei Keimblätter Mesoderm, Entoderm und Ektoderm. Die Zellen können sowohl als organoide Körper als auch als einzelne Zellen gelagert und kultiviert werden und behalten ihre Pluripotenz.

### BEISPIEL 5

Für die Induktion der Differenzierung wurden vorzugsweise Stammzellen nach dem 42. Tag der Kultivierung verwendet. Die Verwendung von Stammzellen nach der 3. oder 4. Passage oder von Zellen, die bei der Temperatur von flüssigem Stickstoff 12-18 Monate lang gelagert worden waren, war ebenfalls problemlos möglich.

Zunächst wurden die Zellen in Differenzierungsmedium mitder oben angegebenen Zusammensetzung überführt und auf eine Dichte von etwa 3 x 10⁴ Zellen/ml eingestellt; z.B. durch Trypsinbehandlung einer Stammzelkultur in Nährmedium, 5-minütige Zentrifugation bei 1000 UpM und Resuspendieren des Pellets in Differenzierungsmedium und Verdünnung soweit erforderlich.

Anschließend wurden mit einer 20-µl Pipette ca. 50 20-µl-Tropfen (600 Zellen/20 µl) auf die Innenseite des Deckels einer bakteriologischen Petrischale gegeben (gestopfte Spitzen) und die Deckel vorsichtig auf die mit PBS gefüllten Petrischalen gestülpt, sodass die Tropfen nach unten hängen. Für jeden Deckel wurde eine neue Spitze verwendet. Die Petrischalen wurden anschließend vorsichtig in den Brutschrank gestellt und 48 h lang bei 37°C inkubiert.

Danach wurden die in den hängenden Tropfen aggregierten Zellen, die organoide Körper (OB), aus jeweils vier Deckeln in je eine bakteriologische Petrischale mit 5 ml Inkubationsmedium mit 20 % FKS überführt (Deckel schräg halten und die OBs mit etwa 2,5 ml Nährmedium abspülen) und für weitere 5-9 Tage, vorzugsweise 96 h, kultiviert.

Die organoiden Körper wurden nun vorsichtig mit einer Pipette aufgesammelt und in mit 0,1 % Gelatine beschichtete Zellkulturgefäße mit Differenzierungsmedium überführt. Die OB vermehrten sich nun und wuchsen in zum Teil einzelnen Zellkolonien, die wieder vermehrt vereinzelt und vermehrt werden konnten. In einer besonders bevorzugten Ausgestaltung des Verfahrens wurden als Kulturgefäß mit 0,1 % Gelatine beschichtete 6 cm Petrischalen verwendet, in die 4 ml Differenzierungsmedium vorgelegt worden war, und diese mit je 6 organoiden Körpern beschickt. Ein weiteres bevorzugtes Kulturgefäß waren mit 0,1 % Gelatine beschichtete Chamber Slides, in die 3 ml Differenzierungsmedium vorgelegt wurde und die anschließend mit je 3-8 organoiden Körpern beschickt wurden und Thermanox-Platten (Nalge Nonc International, USA) für elektronenmikroskopische Studien. Ein weitere Alternative waren 24-Mulden-Mikrotiterplatten, die mit 0,1 % Gelatine beschichtet wurden und in die je 1,5 ml pro Mulde Differenzierungsmedium vorgelegt wurde und die anschließend mit je 4 organoiden Körpern beschickt wurden.

In einer bevorzugten Ausgestaltung des Verfahrens wurden OBs etwa 7 Wochen lang in den gelatine-beschichteten 6-cm-Petrischalen kultiviert und danach wurden einzelne OBs mit dem Microdissector (Eppendorf, Hamburg, Deutschland nach den Anweisungen des Herstellers ausgeschnitten und dann z.B. auf frische 6-cm-Petrischalen, Chamber Slides oder Thermanox-Platten überführt.

### (II) Präparation der Materialzusammensetzung aus den organoiden Körpern und/oder Gewebekörpern

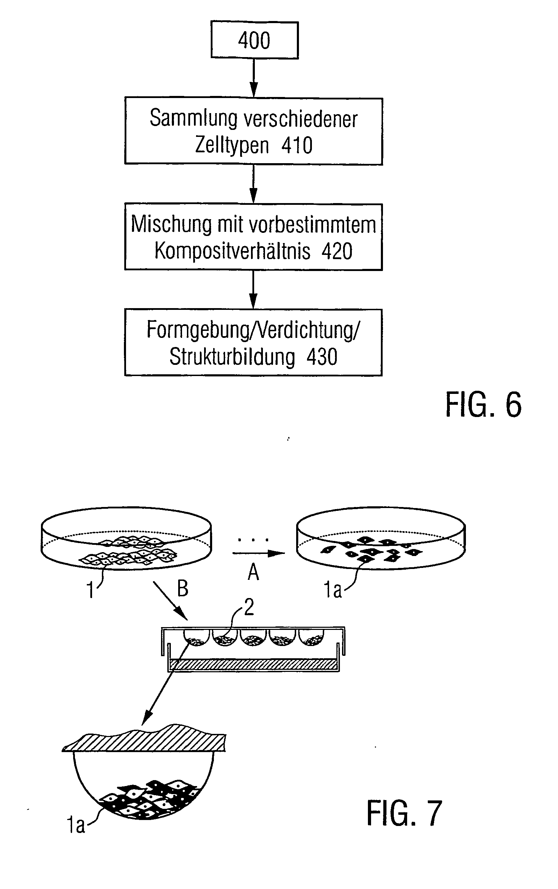

Allgemein können die Schritte der Sammlung und weiteren Bearbeitung (Schritt 400) die in Figur 6 gezeigten Teilschritte umfassen. Gemäß Teilschritt 410 erfolgt die Sammlung ("Ernten") von organoiden Körpern oder Gewebekörpern mit ggf. verschieden differenzierten Zelltypen (Schritt 410). Anschließend werden die verschiedenen Zelltypen in dem gewünschten Kompositverhältnis gemischt (Schritt 420). Schließlich erfolgt die Formgebung, Verdichtung und/oder Strukturbildung am Verbund (Schritt 430).

Die Bereitstellung der verschieden differenzierten Zelltypen ist beispielhaft in Figur 7 gezeigt. Bei dieser Variante des erfindungsgemäßen Verfahrens werden die organoiden Körper 2 unter der Wirkung vordifferenzierter Einzelzellen einer Differenzierung unterzogen. Hierzu werden aus den primär isolierten Stammzellen 1 zunächst differenzierte Stammzellen la gebildet (Pfeil A). Die differenzierten Stammzellen la umfassen zum Beispiel Muskelzellen. Parallel werden aus den primären isolierten Stammzellen 1 entsprechend den oben genannten Prinzipien in hängenden Tropfen organoide Körper 2 gebildet (Pfeil B). Die Muskelzellen la werden in die hängenden Tropfen zugefügt (siehe vergrößertes Teilbild), wo sie eine Differenzierung der organoiden Körper 2 zu Muskelzellen auslösen.

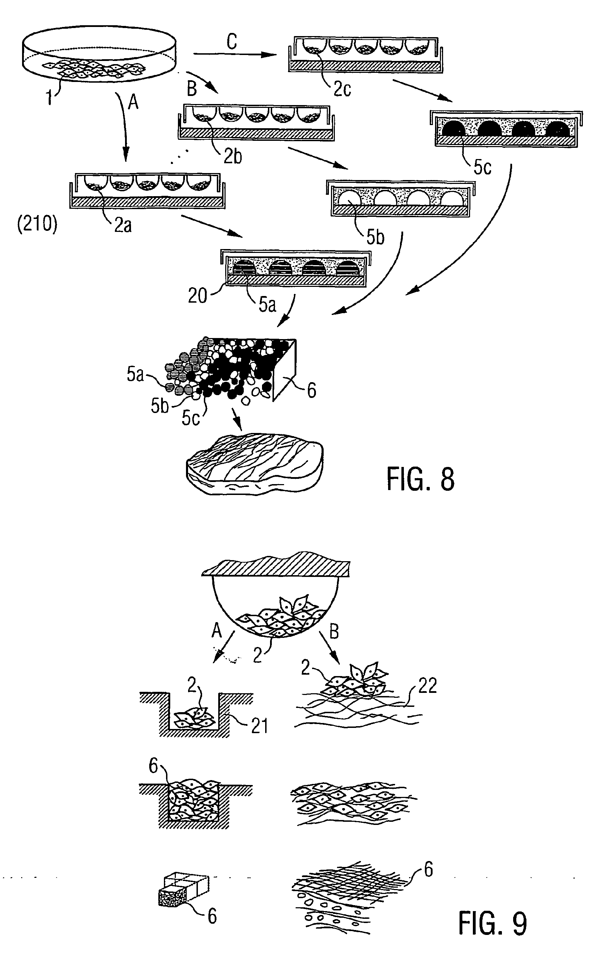

Ein Verfahrensweg zur erfindungsgemäßen Erzeugung einer fleischartigen Gewebezusammensetzung 6 ist schematisch in Figur 8 illustriert. Die primär isolierten Stammzellen 1 umfassen eine wiederholt vermehrbare, nach den oben beschriebenen Prinzipien gewonnene Stammzellenkultur mit pluripotenten Zellen. Ausgehend von den primär isolierten Stammzellen 1 erfolgt zunächst die Bereitstellung von Suspensionskulturen und die Kultivierung im hängenden Tropfen (siehe Figur 3, Schritt 210). In Figur 8 sind beispielhaft drei Suspensionskulturen gezeigt, die im weiteren Verfahren zu verschiedenen Zelltypen führen, die am Ende zu dem gewünschten Komposit 6 zusammengefügt werden sollen. In der ersten Suspensionskultur (Pfeil A) werden bspw. organoide Körper 2a aus Muskelzellen erzeugt, während in den übrigen Suspensionskulturen (Pfeile B und C) z. B. organoide Körper 2b aus Fettzellen und 2c aus Bindegewebezellen erzeugt werden. Auf jedem der Pfade A, B und C werden jeweils einige bis zu einigen Hundert oder sogar einigen Tausend Stammzellen 1 in die hängende Tropfenkultur überführt. Zur Bildung der Tropfenkultur werden verschiedene Kultivierungsmedien verwendet, die zum Beispiel molekulare Differenzierungsfaktoren oder differenzierte Zellen (siehe Figur 7) enthalten. Die Tropfengröße wird durch die Schaffung einer gesättigten Atmosphäre in der Umgebung der Tropfenkultur oder in an sich bekannter Weise durch die Zuführung eines weiteren Kulturmediums konstant gehalten.

Nachdem die organoiden Körper 2a, 2b, 2c gebildet sind und einen Durchmesser von z. B. einigen Hundert Mikrometern besitzen, werden die organoiden Körper in die Kulturschalen 20 in Adhäsionskulturen überführt. Dort erfolgt ein weiteres Wachstum zu Gewebekörpern 5a, 5b und 5c. Da die Kultivierungsmedien in den Kulturschalen 20 verschiedene Signalfaktoren enthalten, besitzen die Gewebekörper 5a, 5b und 5c entsprechend differenziertes Gewebe. Alternativ kann ein Wachstum mit einem Zelltypengemisch vorgesehen sein.

Wenn die Gewebekörper 5a, 5b und 5c eine Größe von z. B. einigen Millimetern erreicht haben, werden Sie gesammelt (Schritt 400, siehe auch Figur 1) und zusammengefügt, so dass ein lockerer Verbund mit einer vorbestimmten quantitativen Zusammensetzung der Gewebekörper 5a, 5b und 5c entsteht. Dieser wird dann mit Hilfe von mechanischen Druckkräften, Ultraschall, mechanischen Vibrationen in die gewünschte Materialzusammensetzung des Komposits 6 überführt. Hierzu werden dem Verbund ggf. Vernetzungsmittel, wie z. B. Gele, Kollagenfasern, Geschmackstoffe, Lebensmittelfarbstoffe oder dgl. zugesetzt.
Das Komposit 6 besitzt typische Dimensionen von beispielsweise einigen 100 µm bis einige mm, kann aber auch größer sein. Bei der Herstellung von Lebensmitteln kann eine Vielzahl von Kompositen zur gewünschten Größe kombiniert werden.

Figur 9 illustriert schematisch die Formgebung des erfindungsgemäß hergestellten biologischen Komposits am Beispiel von verschiedenen Verfahrensvarianten.

Gemäß einer ersten Variante (Pfeil A) werden eine Vielzahl von organoiden Körper 2 oder entsprechend gewachsenen Gewebekörper in eine Prägeeinrichtung eingelegt, die bei diesem Beispiel durch ein Kultivierungssubstrat 21 mit einer vorgegebenen Oberflächenstruktur gebildet wird. Auf dem strukturierten Kultivierungssubstrat 21 erfolgt ein Wachstum und die Vermehrung des Zellmaterials. Dabei kann durch Oberflächenimmobilisierte, molekulare Wachstumsfaktoren (z. B. Proteine) eine bestimmte Differenzierung während des Zellwachstums induziert werden. Wenn die Oberflächenstruktur des Kultivierungssubstrats 21 ausgefüllt ist, kann das Komposit 6 entnommen werden und ggf. weiter geformt oder mit anderen Kompositen zusammengesetzt werden (Teilbild links unten).

Bei der alternativen Variante (Pfeil B) werden die organoiden Körper auf ein Trägersubstrat 22 in Form einer dreidimensio-nalen Matrize aufgebracht. Diese besteht aus verdaubaren Stoffen, wie z. B. Polymeren, Biopolymeren oder dgl.. Beim Wachstum wandern die Zellen des organoiden Körpers 2 in die Trägereinrichtung 22 ein. Entsprechend können mit bandstreifen- oder schichtförmigen Trägereinrichtung verschiedene Grundbausteine geschaffen werden, die zu einem größeren Komposit 6 zusammenfügbar sind (Teilbild rechts unten).

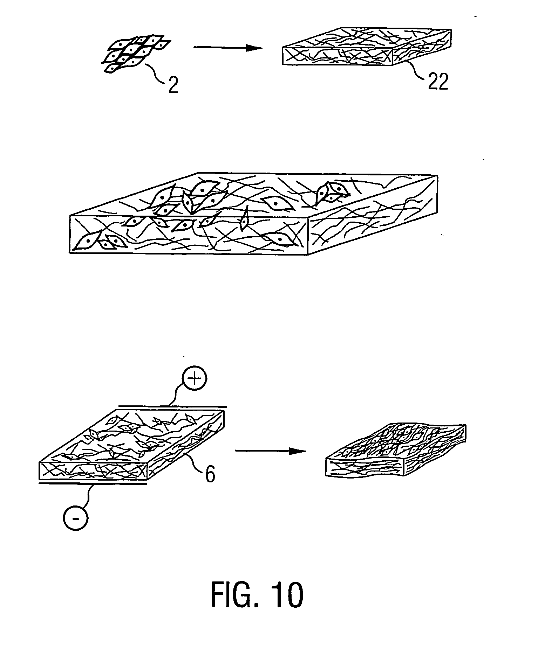

Figur 10 zeigt beispielhaft die Struktureinstellung an einem schichtförmigen Trägersubstrat 22, die analog zur Variante B in Figur 9 mit organoiden Körpern oder Gewebekörpern bewachsen ist, die vorrangig aus Muskelzellen bestehen. Wenn das Trägersubstrat 22, das bspw. ein dreidimensionales Kollagen-Vlies aus autologem Kollagen umfasst, vollständig durchdrungen und bewachsen ist, wird das Komposit 6 einem Strompuls ausgesetzt. Daraufhin kontrahieren die Muskelzellen im Komposit 6 entgegen den Kräften der Kollagenmatrix. Es wird eine innere Spannung eingestellt, die mit einer erhöhten Festigkeit des Komposits verbunden ist.

Die in der vorstehenden Beschreibung, den Ansprüchen und den Zeichnungen offenbarten Merkmale der Erfindung können sowohl einzeln oder auch in Kombination für die Verwirklichung der Erfindung in ihren verschiedenen Ausgestaltungen von Bedeutung sein.

## Patentansprüche

1. Verfahren zur Herstellung einer biologischen Materialzusammensetzung tierischen, nicht-menschlichen Ursprungs, insbesondere als Futtermittel, umfassend:
- Aggregation von pluripotenten Stammzellen aus differenziertem exokrinen Drüsengewebe eines tierischen Organismus zu organoiden Körpern, und
- Präparation der Materialzusammensetzung aus den organoiden Körpern, wobei die Präparation das Zusammenfügen von organoiden Körpern oder daraus durch Wachstum entstandenen Gewebekörpern zu einem Komposit umfasst, und wobei die Bildung und Formgebung des Komposits geschieht durch
a) das Einlegen einer Vielzahl von organoiden Körper oder Gewebekörpern in eine Prägeeinrichtung, die durch ein Kultivierungssubstrat mit einer vorgegebenen Oberflächenstruktur gebildet wird, und das Wachstum und die Vermehrung des Zellmaterials auf dem Kultivierungssubstrat bis die Oberflächenstruktur des Kultivierungssubstrats ausgefüllt ist, oder
b) das Aufbringen der organoiden Körper auf eine Trägereinrichtung in Form einer dreidimensionalen Matrize aus verdaubaren Stoffen, wobei die Zellen der organoiden Körper beim Wachstum in die Trägereinrichtung einwandern.

2. Verfahren nach Anspruch 1, bei dem die Aggregation der Stammzellen in einem Kulturmedium ohne einen Zusatz erfolgt, der eine Differenzierung von Zellen beeinflusst.

3. Verfahren nach Anspruch 1, bei dem die Aggregation der Stammzellen in einem Kulturmedium erfolgt, das mindestens einen Zusatz enthält, der eine Differenzierung der Stammzellen bei der Aggregation beeinflusst.

4. Verfahren nach mindestens einem der vorhergehenden Ansprüche, bei dem die Aggregation von Stammzellen zu primären organoiden Körpern führt, wobei die Präparation der Materialzusammensetzung eine Bildung von sekundären organoiden Körpern aus den primären organoiden Körpern umfasst.

5. Verfahren nach mindestens einem der vorhergehenden Ansprüche, bei dem die Präparation der Materialzusammensetzung ein Wachstum der organoiden Körper zu Gewebekörpern umfasst.

6. Verfahren nach mindestens einem der vorhergehenden Ansprüche 3-5, bei dem die Differenzierung durch einen Zusatz von differenzierten Zellen beeinflusst wird.

7. Verfahren nach Anspruch 6, bei dem die Differenzierung durch einen Zusatz von differenzierten autologen Zellen beeinflusst wird.

8. Verfahren nach mindestens einem der Ansprüche 1-7, bei dem die Stammzellen aus Drüsengewebe eines Wirbeltieres isoliert werden.

9. Verfahren nach Anspruch 8, bei dem die Stammzellen aus Drüsengewebe eines Fisches, eines Vogels oder eines nicht-menschlichen Säugetieres isoliert werden.

## Claims

1. A method for producing a biological material composition of animal, non-human origin, especially as feed, comprising:
- an aggregation of pluripotent stem cells from differentiated exocrine glandular tissue of an animal organism to organoid bodies, and
- a preparation of the material composition from the organoid bodies, wherein the preparation comprises combining of the organoid bodies or of tissue bodies generated therefrom by growth to a composite and wherein the production and shaping of the composite is effected by
a) placing of a plurality of organoid bodies or tissue bodies in an imprinting device formed by a cultivation substrate with a given surface structure, and growth and propagation of the cell material on the cultivation substrate until the surface structure of the cultivation substrate is filled, or
b) placing the organoid bodies on a carrier device in the form of a three dimensional matrix consisting of digestible substances, wherein the cells of the organoid bodies migrate into the carrier device during the growth.

2. The method according to claim 1, in which the aggregation of the stem cells takes place in a culture medium without an additive that influences a differentiation of cells.

3. The method according to claim 1, wherein the aggregation of the stem cells takes place in a culture medium containing at least one additive that influences a differentiation of these stem cells during the aggregation.

4. The method according to at least one of the preceding claims, wherein the aggregation of stem cells results in primary organoid bodies, the preparation of the material composition comprising a formation of secondary organoid bodies from the primary organoid bodies.

5. The method according to at least one of the preceding claims, wherein the preparation of the material composition comprises a growth of the organoid bodies to tissue bodies.

6. The method according to at least one of the preceding claims 3-5, in which the differentiation is influenced by an addition of differentiated cells.

7. The method according to claim 6, in which the differentiation is influenced by an addition of autologous cells.

8. The method according to at least one of claims 1-7, in which the stem cells are isolated from glandular tissue of a vertebrate.

9. The method according to claim 8, in which the stem cells are isolated from glandular tissue of a fish, bird or a non-human mammal.

## Revendications

1. Procédé de préparation d'une composition de matière biologique d'origine animale, non humaine, en particulier sous forme d'alimentation animale, comprenant les étapes de :
- agrégation de cellules souches pluripotentes provenant de tissus différencié de glandes exocrines d'un organisme animal en corps organoïdes, et
- préparation de la composition de matière à partir des corps organoïdes, la préparation comprenant le rassemblement des corps organoïdes ou des corps tissulaires qui en résultent par croissance en un composite, et la formation et le formage du composite se faisant par :
a) l'insertion d'une multitude de corps organoïdes ou de corps tissulaires dans un dispositif de gaufrage, qui est constitué par un substrat de culture ayant une structure de surface prédéterminée, et la croissance et la multiplication de la matière cellulaire sur le substrat de culture jusqu'à ce que la structure de surface du substrat de culture soit entièrement remplie, ou
b) le dépôt des corps organoïdes sur un dispositif support prenant la forme d'une matrice tridimensionnelle constituée de matières digestes, les cellules des corps organoïdes migrant dans le dispositif support lors de la croissance.

2. Procédé selon la revendication 1, dans lequel l'agrégation des cellules souches dans un milieu de culture se fait sans recourir à une addition qui influence une différenciation des cellules.

3. Procédé selon la revendication 1, dans lequel l'agrégation des cellules souches s'effectue dans un milieu de culture qui comprend au moins un additif, qui influence une différenciation des cellules souches lors de l'agrégation.

4. Procédé selon au moins une des revendications précédentes, dans lequel l'agrégation des cellules souches donne des corps organoïdes primaires, la préparation de la composition de matière comprenant une formation de corps organoïdes secondaires à partir des corps organoïdes primaires.

5. Procédé selon au moins une des revendications précédentes, dans lequel la préparation de la composition de matière comprend une croissance des corps organoïdes en corps tissulaires.

6. Procédé selon au moins une des revendications précédentes 3 à 5, dans lequel la différenciation est influencée par une addition de cellules différenciées.

7. Procédé selon la revendication 6, dans lequel la différenciation est influencée par une addition de cellules différenciées autologues.

8. Procédé selon au moins une des revendications précédentes 1 à 7, dans lequel les cellules souches sont isolées à partir du tissu de glandes d'un vertébré.

9. Procédé selon la revendication 8, dans lequel les cellules souches sont isolées à partir du tissu de glandes d'un poisson, d'un oiseau ou d'un mammifère non humain.
